# EUROPEAN PATENT SPECIFICATION

(11) **EP 3 395 350 B1**
(45) Date of publication and mention of the grant of the patent: **15.06.2022**
(21) Application number: 16879037.6
(22) Date of filing: 26.12.2016
(51) Int. Cl.: A61K 35/28, A61K 38/49, A61P 9/08, A61P 7/02, A61P 9/10

(54) **MEDICAL DRUG FOR TREATING CEREBRAL INFARCTION**
ARZNEIMITTEL ZUR BEHANDLUNG EINES HIRNINFARKTS
MÉDICAMENT POUR LE TRAITEMENT DES INFARCTUS CÉRÉBRAUX

(30) Priority: 25.12.2015 JP 2015254410
(43) Date of publication of application: 31.10.2018
(73) Proprietor: Sapporo Medical University, Sapporo-shi, Hokkaido 060-8556 (JP); Nipro Corporation, Osaka-shi, Osaka 531-8510 (JP)
(72) Inventor: HONMOU Osamu, Sapporo-shi Hokkaido 060-8556 (JP); NAKAZAKI Masahito, Sapporo-shi Hokkaido 060-8556 (JP); SASAKI Masanori, Sapporo-shi Hokkaido 060-8556 (JP); SASAKI Yuko, Sapporo-shi Hokkaido 060-8556 (JP); OKA Shinichi, Sapporo-shi Hokkaido 060-8556 (JP)
(74) Representative: Hoffmann Eitle
(86) International application number: PCT/JP2016/088646
(87) International publication number: WO 2017/111153

(56) References cited:
- WO-A1-2005/007176
- WO-A1-2015/046574
- WO-A1-2015/174087
- WO-A2-2004/044142
- JP-A- 2012 100 662
- JP-A- 2013 542 178
- LI SHU-JUN ET AL: "Targeted introduction of tissue plasminogen activator (TPA) at the AAVS1 locus in mesenchymal stem cells (MSCs) and its stable and effective expression", BIOCHEMICAL AND BIOPHYSICAL RESEARCH COMMUNICATIONS, ELSEVIER, AMSTERDAM, NL, vol. 437, no. 1, 20 June 2013 (2013-06-20) , pages 74-78, XP028679473, ISSN: 0006-291X, DOI: 10.1016/J.BBRC.2013.06.037
- ZHU ZHAOHUI ET AL: "MSC treatment of cerebral hemorrhage: Evaluation of monkey models with serial FDG PET", THE JOURNAL OF NUCLEAR MEDICINE, SOCIETY OF NUCLEAR MEDICINE, US, vol. 50, no. Suppl. 2, 30 April 2009 (2009-04-30), page 20, XP009514853, ISSN: 0161-5505
- MAY-JYWAN TSAI ET AL: "Recovery of neurological function of ischemic stroke by application of conditioned medium of bone marrow mesenchymal stem cells derived from normal and cerebral ischemia rats", JOURNAL OF BIOMEDICAL SCIENCE, KLUWER ACADEMIC PUBLISHERS, DO, vol. 21, no. 1, 22 January 2014 (2014-01-22), page 5, XP021175334, ISSN: 1423-0127, DOI: 10.1186/1423-0127-21-5
- LIAO LI ET AL: "Heparin improves BMSC cell therapy: Anticoagulant treatment by heparin improves the safety and therapeutic effect of bone marrow-derived mesenchymal stem cell cytotherapy", THERANOSTICS, vol. 7, no. 1, 2017, pages 106-116, XP009514938,
- JUN'ICHI KAWABE: "Tokushu Kessensho ni Kansuru Q&A PART6, 8. Yakuzai, Q57 Prostacycline to Kekkan Saisei ni Tsuite Oshiete Kudasai", Thrombosis and Circulation, vol. 19, no. 1, 2011, pages 189-191, XP9512769,
- ATSUSHI NAGAI et al.: "Tokushu Rinsho eno Kanosei o Saguru Nosocchu Saisei Iryo no Saisentan, Kotsuzui Kan'yokei Kansaibo ni yoru Noshukketsu Saisei Iryo", Molecular cerebrovascular medicine, vol. 5, no. 4, 2006, pages 33-39, XP009512068, ISSN: 1346-8995
- LIU, J. et al.: "The Repairing Effect of Human Mesenchymal Stem Cells(hMSCs) on Cynomlgus Macaque with intracerebral hemorrhage", Experimental animals, vol. 58, no. 3, 2009, page 232, XP009511309, ISSN: 1341-1357

## Description

### Technical Field

### [Technical Field]

The present invention relates to a medicament for treating an ischemic vascular disorder, comprising mesenchymal stem cells as defined in the appended claim.More specifically, the present invention relates to a medicament for treating an ischemic vascular disorder that reduces risk of cerebral hemorrhage associated with a reperfusion therapy for vessel occlusion such as thrombolytic therapy and can extend the therapeutic time window as defined in the appended claims.

### Background Art

Cerebral infarction refers to a pathological condition in which cerebral ischemia occurs due to cerebral artery occlusion or stenosis and brain tissue undergoes necrosis or a similar state. For cerebral infarction (in particular an acute phase or super acute phase of cerebral infarction) or myocardial infarction, thrombolysis or physical removal of a blood clot is the first-line choice for the treatment to prevent necrosis due to ischemia. However, the reperfusion for vessel occlusion has a problem that it is often associated with a risk of cerebral hemorrhage.

The t-PA IV therapy has higher thrombolysis activity and lower risk of hemorrhage than other thrombolytic agents such as urokinase and is therefore the standard of care in a reperfusion therapy of a super acute phase or acute phase of cerebral infarction. However, since the therapeutic effect of t-PA decreases and the risk of hemorrhage increases over time, the therapy must usually start within 4.5 hours from the onset. Therefore, a new therapy that suppresses endothelial dysfunction is demanded in order to reduce the risk of hemorrhage and to extend the therapeutic time window for t-PA.

Meanwhile, mesenchymal stem cells (MSCs) are known to provide the protection of brain (parenchyma and blood vessel). It is confirmed using an experimental infarction model that the MSC administration after cerebral infarction improves the behavioral function and reduces the ischemic lesion volume (Non Patent Literature 1 to 3, Patent Literature 1). Moreover, the treatment of cerebral infarction patients by intravenous administration of MSCs have been conducted many times and the improvement of motor function and lesion has been reported (Non Patent Literature 4, Patent Literature 2). Li, Shu-Jun, et al. describe targeted introduction of tissue plasminogen activator (TPA) at the AAVS1 locus in mesenchymal stem cells (MSCs) and its stable and effective expression (Li, Shu-Jun, et al. "Targeted introduction of tissue plasminogen activator (TPA) at the AAVS1 locus in mesenchymal stem cells (MSCs) and its stable and effective expression." Biochemical and biophysical research communications 437.1 (2013): 74-78

### Citation List

### Patent Literature

Patent Literature 1: WO2002/000849
Patent Literature 2: WO2009/002503

### Non Patent Literature

Non Patent Literature 1: Iihoshi S. et al., Brain Res. 2004, 1007: 1-9.
Non Patent Literature 2: Nomura T. et al., Neuroscience. 2005, 136: 161-169.
Non Patent Literature 3: Honma T. et al., Exp. Neurol. 2006, 199: 56-66.
Non Patent Literature 4: Honmou O. et al., Brain. 2011, 134: 1790-1807.

### Summary of Invention

### Technical Problem

An object of the present invention is to provide a new method for treating cerebral hemorrhage associated with a reperfusion therapy for vessel occlusion and, in particular, to provide means for reducing risk of cerebral hemorrhage associated with thrombolytic therapy and treating motor dysfunction and higher brain dysfunction associated with an ischemic vascular disorder.

### Solution to Problem

The inventors have found that by intravenously administering MSCs, in combination with thrombolytic therapy with rt-PA, to a transient middle cerebral artery occlusion model that has been induced by inserting an intraluminal suture into the artery, hemorrhagic infarction is suppressed markedly and significant improvement of motor function over time is exhibited.

The present invention is defined in the appended claims.

Also disclosed is the following:

Accordingly, the present disclosure relates to the following (1) to (18).
(1) A medicament for treating an ischemic vascular disorder, comprising mesenchymal stem cells, wherein the medicament is used in combination with a reperfusion therapy for vessel occlusion;
(2) the medicament according to (1) above, wherein the medicament reduces risk of cerebral hemorrhage associated with the reperfusion therapy for vessel occlusion;
(3) the medicament according to (1) or (2) above, wherein the reperfusion therapy for vessel occlusion comprises any one or more selected from administration of a thrombolytic agent, a platelet aggregation inhibitor, and an anticoagulant agent, and physical removal of a blood clot;
(4) the medicament according to any one of (1) to (3) above, wherein the reperfusion therapy for vessel occlusion comprises administration of the thrombolytic agent;
(5) the medicament according to (4) above, wherein the thrombolytic agent is plasminogen activator (t-PA);
(6) the medicament according to any one of (3) to (5) above, wherein the medicament extends a time window in which the thrombolytic agent can be administered;
(7) the medicament according to any one of (1) to (6) above, wherein the ischemic vascular disorder is an ischemic cerebrovascular disorder or myocardial infarction;
(8) the medicament according to any one of (1) to (6) above, wherein the ischemic vascular disorder is a super acute phase or acute phase of an ischemic cerebrovascular disorder or acute myocardial infarction;
(9) the medicament according to any one of (1) to (8) above, wherein the mesenchymal stem cells are mesenchymal stem cells derived from bone marrow;
(10) a medicament for preventing cerebral hemorrhage associated with a reperfusion therapy for vessel occlusion, comprising mesenchymal stem cells.
(11) the medicament according to (10) above, wherein the reperfusion therapy for vessel occlusion comprises any one or more selected from administration of a thrombolytic agent, a platelet aggregation inhibitor, and an anticoagulant agent, and physical removal of a blood clot;
(12) the medicament according to (10) or (11) above, wherein the reperfusion therapy for vessel occlusion comprises administration of the thrombolytic agent;
(13) the medicament according to (12) above, wherein the thrombolytic agent is plasminogen activator;
(14) the medicament according to any one of (11) to (13) above, wherein the medicament extends a time window in which the thrombolytic agent can be administered;
(15) the medicament according to any one of (10) to (14) above, wherein the medicament is used for a patient with an ischemic vascular disorder;
(16) the medicament according to (15) above, wherein the ischemic vascular disorder is an ischemic cerebrovascular disorder or myocardial infarction;
(17) the medicament according to (15) or (16) above, wherein the ischemic vascular disorder is a super acute phase or acute phase of an ischemic cerebrovascular disorder or myocardial infarction;
(18) the medicament according to any one of (10) to (17) above, wherein the mesenchymal stem cells are mesenchymal stem cells derived from bone marrow.

### Advantageous Effects of Invention

According to the present invention, a synergistic effect of reducing risk of cerebral hemorrhage associated with a reperfusion therapy for vessel occlusion such as t-PA therapy and improving a therapeutic effect (improvement of motor function, decrease in infarction lesion) of MSCs is accomplished. Moreover, by the reduction of risk of cerebral hemorrhage, the time window in which a thrombolytic therapy such as t-PA therapy can be applied, which is usually considered to be 4.5 hours after the onset, can be extended.

### Brief Description of Drawings

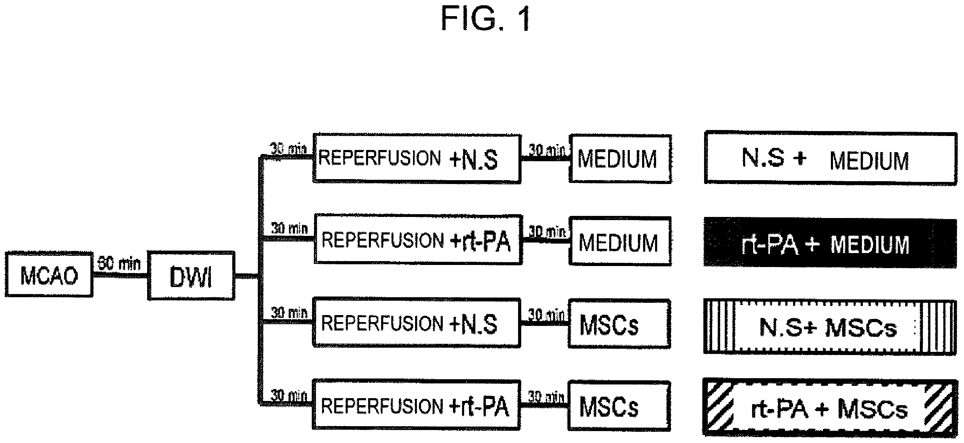

[Figure 1] Figure 1 illustrates the outline of the experimental protocol. DWI is acquired 60 minutes after middle cerebral artery occlusion (MCAO); after grouping into 4 groups at random, occlusion is recanalized and rt-PA or physiological saline is administered (90 minutes after occlusion); and a medium or MSC is administered further after 30 minutes.

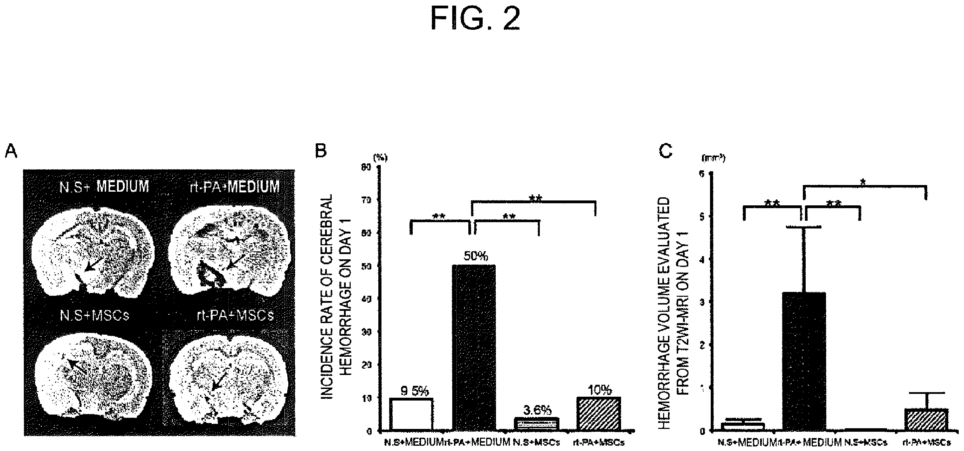

[Figure 2] Figure 2 illustrates the onset of acute hemorrhage after the combination of rt-PA therapy and MSC administration. A: Images of respective groups (physiological saline + medium, rt-PA + medium, physiological saline + MSC, rt-PA + MSC), B: Incidence rate of cerebral hemorrhage on Day 1, C: Hemorrhage volume evaluated from T2WI-MRI on Day 1 (** P < 0.01, * P < 0.05, Tukey's post-hoc test).

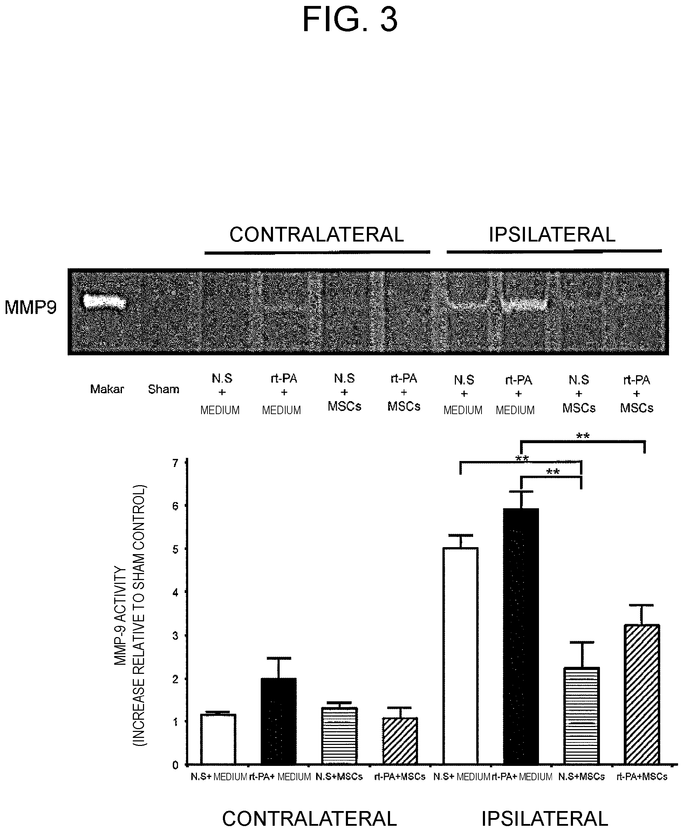

[Figure 3] Figure 3 illustrates a result of gelatin zymography. A: Expression level of MMP-9 (from the left, Marker, Sham, Contralateral: physiological saline + medium, rt-PA + medium, physiological saline + MSC, rt-PA + MSC, Ipsilateral: physiological saline + medium, rt-PA + medium, physiological saline + MSC, rt-PA + MSC). B: MMP-9 activity by densitometry measurement (** P < 0.01, Tukey's post-hoc test).

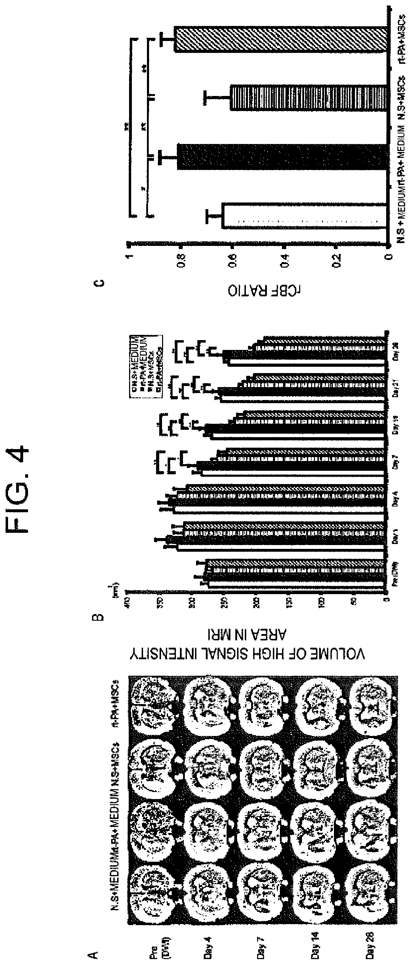

[Figure 4] Figure 4 illustrates characteristics of ischemia lesion by MRI analysis. A: DWI before occlusion (Column 1), T2WI on Day 4 (Column 2), Day 7 (Column 3), Day 14 (Column 4), Day 28 (Column 5) after reperfusion in respective groups. B: Lesion volumes of respective groups evaluated from DWI (before occlusion) and T2WI of Day 1, 4, 7, 14, 21, 28 after reperfusion. C: rCBF evaluated from PWI (** P < 0.01, * P < 0.05, Tukey's post-hoc test).

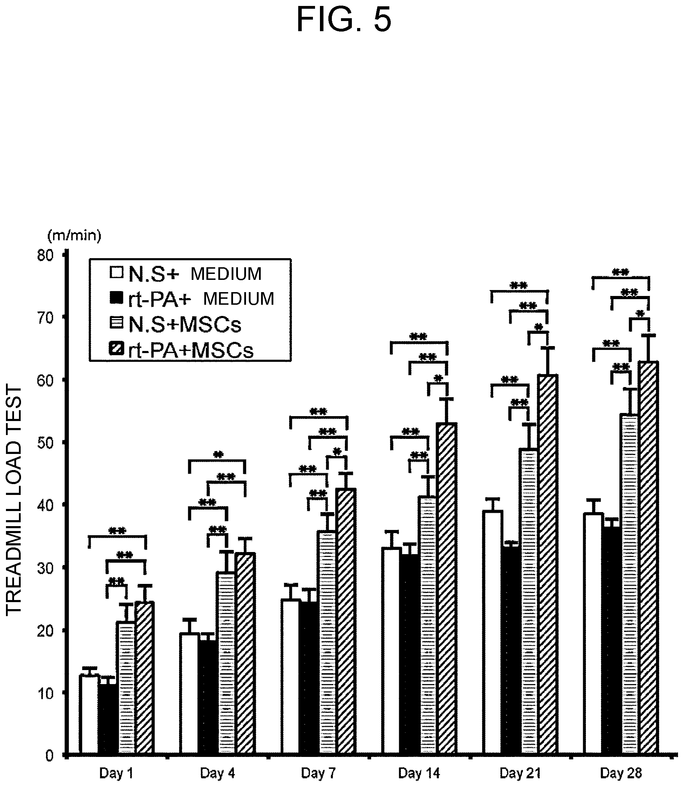

[Figure 5] Figure 5 illustrates a result of treadmill load test. Maximum speeds at which rats were able to run over a treadmill on Day 1, Day 4, Day 7, Day 14, Day 21, Day 28 after reperfusion in respective groups (** P < 0.01, * P < 0.05, Tukey's post-hoc test).

### Description of Embodiments

### 1. Medicament for treating ischemic vascular disorder

The present invention relates to a medicament for treating an ischemic vascular disorder, comprising mesenchymal stem cells, wherein the medicament is used in combination with a reperfusion therapy for vessel occlusion comprising administration of a tissue plasminogen activator.

### [Mesenchymal stem cell]

Mesenchymal stem cells used in the present invention are stem cells having multipotency and self-renewal present in a very small amount in stroma cells of mesenchymal tissue and known to not only differentiate into connective tissue cells such as osteocytes, chondrocytes, and adipocytes, but also have differentiation potency into neural cells and cardiomyocytes.

Sources of the mesenchymal stem cells include bone marrow, peripheral blood, umbilical cord blood, fetal embryos, and brain, but are preferably mesenchymal stem cells derived from bone marrow (bone marrow mesenchymal stem cells), in particular, human bone marrow mesenchymal stem cells. The mesenchymal stem cells derived from bone marrow have advantages in that 1) a marked effect can be expected, 2) risk of side effects is low, 3) sufficient supply of donor cells can be expected, and 4) therapies with them are noninvasive and they can be autografted and therefore 5) risk of infection is low, 6) immunorejection does not need to be worried about, 7) they have no ethical problems, 8) they are easy to be accepted socially, and 9) they are easy to become a therapy widely used as a general medical care. Furthermore, the bone marrow transplantation therapy is a therapy already used on the clinical site and its safety is confirmed. Moreover, stem cells derived from bone marrow are highly migratory and not only by the transplant to the local site but also by intravenous administration, they can be delivered to lesional tissue and a therapeutic effect can be expected there.

The cells may be cells obtained by inducing differentiation of ES cells or induced pluripotent stem cells (iPS cells or the like), an established cell line, or cells isolated from the living body and proliferated. The cells may be derived from allogeneic cells or autologous cells, but they are preferably mesenchymal stem cells derived from autologous cells (derived from patient's own cells).

The mesenchymal stem cells used in the present invention are preferably in an undifferentiated state. This is because cells in an undifferentiated state have a high reproductive rate and a high survival rate after the introduction into the living body. The inventors have developed a method for obtaining such cells, details of which are described in WO2009/002503.

In the aforementioned method developed by the inventors, cells separated from a bone marrow aspirate under conditions in which the cells do not come in substantial contact with an anticoagulant (heparin) are proliferated in a medium containing human serum (preferably autologous serum) and containing no anticoagulant (heparin) or an anticoagulant at a very low concentration.

The density of the cells in the medium has an effect on properties and the direction of differentiation of the cells. In the case of mesenchymal stem cells, cell densities in a medium higher than 8,500 cells/cm² change the properties of the cells and therefore it is preferred to passage the cells at a cell density lower than or at most equal to 8500/cm² and it is more preferable to passage the cells at a time point when the cell density become equal to or higher than 5500/cm².

In the aforementioned method that the inventors developed, a human serum-containing medium is used and therefore, in consideration of the burden on the serum donor, it is desirable that the number of the medium change is as little as possible and, for example, the medium change is conducted at least once a week and more preferably 1 to 2 times a week.

In the culture, the cells are repeatedly passaged until the total number of the cells reaches 10⁸ or more. The number of required cells may vary depending on the purpose of use, but for example, the number of mesenchymal stem cells required for the transplant for treating an ischemic brain disease such as cerebral infarction is considered to be equal to or higher than 10⁷. According to the method that inventors developed, 10⁷ mesenchymal stem cells can be obtained in about 12 days.

The proliferated MSCs may be stored by techniques such as the cryopreservation (for example, in a deep freezer at -152 degrees Celsius) until use as needed. In cryopreservation, a medium (a medium for mammalian cells such as RPMI) is used as a cryopreservation medium containing serum (preferably human serum, more preferably autologous serum), dextran, DMSO. For example, cells can be suspended in a cryopreservation medium containing 20.5 mL of RPMI sterilized by usual filtration, 20.5 mL of self-serum collected from a patient, 5 mL of dextran, and 5mL of DMSO and cryopreserved at -150 degrees Celsius. Examples of DMSO and dextran include, but are not limited to, Cryoserv made by Nipro Corporation and Low Molecular Dextran L Injection made by Otsuka Pharmaceutical Co., Ltd., respectively.

The higher number of the MSCs contained in the medicament according to the present invention is, the more preferable it is. However, it is practical to be the minimum number at which the MSCs are effective in consideration of the timing at which they are administered to a subject and the time required for the culture. Accordingly, in a preferred aspect of the medicament according to the present invention, the number of mesenchymal stem cells is 10⁷ or more, preferably 5 × 10⁷ or more, more preferably 10⁸ or more, further preferably 5 × 10⁸ or more.

The medicament according to the present invention is preferably a formulation for parenteral administration, more preferably a formulation for parenteral systemic administration, and particularly a formulation for intravenous administration. Examples of the dosage form suitable for the parenteral administration include injections such as solution-type injections, suspension-type injections, emulsion-type injections, and injections prepared at time of use and grafts. The formulation for parenteral administration is in the form of an aqueous or nonaqueous isotonic aseptic solution or suspension, is formulated into an appropriate unit dosage form in combination with, for example, a pharmacologically acceptable carrier or vehicle, such as, specifically, sterile water or physiological saline, a medium (medium particularly used in the culture of mammalian cells, such as RPMI), a physiological buffer solution such as PBS, a vegetable oil, an emulsifier, a suspension, a surfactant, a stabilizer, an excipient, a vehicle, a preservative, a binder, or the like, as appropriate.

Examples of an aqueous solution for injection include physiological buffer solutions such as physiological saline, a medium, and PBS, isotonic solutions containing glucose or another pharmaceutic aid, for example, D-sorbitol, D-mannose, D-mannitol, sodium chloride, or the like, which may be used in combination with a suitable solubilizing agent, for example, alcohol, more specifically; ethanol, polyalcohol, propylene glycol, polyethyleneglycol, and non-ionic surfactants, for example, polysorbate 80, HCO-50, or the like.

### [Ischemic vascular disorder]

The medicament according to the present invention is used to treat an ischemic vascular disorder. The ischemic vascular disorder refers to a condition that presents a local vascular disorder (for example, degeneration or occlusion) due to a decrease in artery bloodstream and examples thereof include ischemic cerebrovascular disorders and ischemic heart diseases.

### [Ischemic cerebrovascular disorder]

The medicament according to the present invention is used to treat an ischemic cerebrovascular disorder. As described above, the MSCs are known to provide the protection of the brain (the parenchyma and the blood vessel) and have been already used in therapies for an ischemic cerebrovascular disorder such as cerebral infarction by intravenous administration.

Examples of the ischemic cerebrovascular disorder include cerebral infarction (for example, atherothrombotic brain infarction, cerebral thrombosis, cerebral embolism, lacunar infarction, branch atheromatous disease (BAD), Trousseau's syndrome, blood coagulation disorder, artery dissection, venous infarction, vasculitis, antiphospholipid antibody syndrome), transient ischemic attack (TIA), and the like.

### [Ischemic heart disease]

The medicament according to the present invention is also used to treat ischemic heart disease such as myocardial infarction. The myocardial infarction refers to a condition in which occlusion or stenosis develops in the coronary blood vessel that supplies oxygen and nutrition to the heart, reducing the blood flow, to make the heart muscle in an ischemic state and necrotized.

The medicament according to the present invention is suitable for the reperfusion therapy for vessel occlusion, particularly a super acute phase (within 8 hours) or acute phase (8 hours to 24 hours) of an ischemic cerebrovascular disorder or acute myocardial infarction (within 3 days from the onset), to which the thrombolytic therapy is applied, but the timing of application is determined as appropriate according to the symptom of the patient.

### [Thrombolytic agent]

For an ischemic vascular disorder (particularly an acute phase or super acute phase of an ischemic cerebrovascular disorder or myocardial infarction), reperfusion of the occluded blood vessel by thrombolysis, physical removal of the blood clot, or the like is the first-line choice for the treatment to prevent necrosis due to ischemia.

Examples of the thrombolytic agent include urokinase, pro-urokinase, tissue plasminogen activator (t-PA), nasaruplase, streptokinase, and the like.

Any t-PA that is used at present is recombinant t-PA (rt-PA). Examples of the rt-PA include alteplase and tisokinase, which have an amino acid sequence same as a native t-PA and also pamiteplase, in which an amino acid modification has been made to extend the half-life, and the like. Medicaments containing t-PA currently in the market in Japan include ACTIVACIN (R) injection, GRTPA
(R) injection, and Cleactor (R) injection, among which the first two are approved for application to an ischemic cerebrovascular disorder and acute myocardial infarction. Moreover, the last one is approved for application to acute myocardial infarction and acute pulmonary embolism.

While thrombolytic agents can improve ischemia by dissolving clots and prevent necrosis of the brain tissue, they are associated with a risk of hemorrhage. Moreover, the timing of administration is limited, for example, to a time window within 6 hours from the onset for urokinase and a time window within 4.5 hours from the onset for t-PA.

Since t-PA activates plasmin, which dissolves fibrin, a main component of blood clots, and thereby has a higher ability to lyse the blood clot by than other thrombolytic agents, it has become the first choice of thrombolytic therapy at present. Moreover, t-PA has both the effects as a platelet aggregation inhibitor and an anticoagulant agent. However, as described above, its application is limited by the risk of cerebral hemorrhage and the short time window of 4.5 hours for administration.

The medicament according to the present invention can reduce the risk of cerebral hemorrhage and extend the time window for the administration the therapeutic time window thereof by being used in combination with a medicament containing t-PA.

### [Platelet aggregation inhibitor (antiplatelet agent)]

The platelet aggregation inhibitor (antiplatelet agent) can prevent the clot formation by inhibiting the aggregation of platelets. Examples of the platelet aggregation inhibitor include, but are not limited to, aspirin, clopidogrel, cilostazol, ticlopidine, and the like.

### [Anticoagulant agent (anticoagulant)]

The anticoagulant agent (anticoagulant) can prevent the clot formation by inhibiting the function of the coagulation factors. Examples of the anticoagulant agent include, but are not limited to, heparin, low molecular weight heparin, argatroban, danaparoid sodium, dalteparin, nadroparin, bemiparin, fondaparinux, antithrombin agents such asargatroban, and the like.

The medicament according to the present invention may be applied before, after, or at the same time as the reperfusion therapy for vessel occlusion. The medicament according to the present invention (MSCs) can easily be delivered to the affected portion by intravenous administration, a simple and easy way.

### [Physical removal of blood clot]

The method for physical removal of a blood clot include, but are not limited to, mechanical clot retrieval therapy by an intravascular operation or the like, carotid endarterectomy, and the like. Moreover, the method may also be reperfusion by a bypass surgery, stent treatment, ballooning, therapeutic aspiration, disruption by supersonic wave, or the like.

### [Synergistic effect]

When used in combination with a reperfusion therapy for vessel occlusion (for example, administration of a medicament including a thrombolytic agent, a platelet aggregation inhibitor, and an anticoagulant agent, or physical removal of a blood clot), the medicament according to the present invention can reduce risk of cerebral hemorrhage associated with the reperfusion therapy for vessel occlusion. In particular, while the application of thrombolytic agents is limited because of its short time window for administration, the medicament according to the present invention can extend the time window in which thrombolytic agents can be administered. For example, by using t-PA in combination with the medicament according to the present invention, the time window for administration of t-PA can be expected to be extended at least 4.5 hours, 6 hours, 8 hours, 12 hours, 24 hours or more.

Furthermore, the thrombolytic agent improves the therapeutic effect of MSCs by improving microcirculation and markedly increases the effect of improving the motor function and the tissue repair effect by MSCs in comparison with that when administered singly. In this way, the combinatory use of a medicament containing a thrombolytic agent and the medicament according to the present invention produces a synergistic effect that cannot be predicted from the single administration.

### 2. Preventive agent for cerebral hemorrhage associated with reperfusion therapy for vessel occlusion

The present invention also provides a medicament for preventing cerebral hemorrhage associated with the reperfusion therapy (comprising administration of a tissue pasminogen activator for vessel occlusion, comprising mesenchymal stem cells. As described above, the reperfusion therapy for vessel occlusion in ischemic vascular disorder is associated with risk of cerebral hemorrhage, but the medicament according to the present invention can suppress matrix metalloproteinase (MMP)-9 activation and thereby suppress the endothelial dysfunction associated therewith and reduce the risk of cerebral hemorrhage.

In particular, the medicament according to the present invention makes it possible to apply thrombolytic therapy to patients whom no thrombolytic therapy has conventionally been able to apply to by preventing cerebral hemorrhage associated with administration of thrombolytic agent which is t-PA and extending the time window for the administration thereof. The medicament according to the present invention can ameliorate motor dysfunction associated with an ischemic cerebrovascular disorder by tissue regeneration and restoration effects that MSCs have and promote healing of infarction lesion and the thrombolytic agent further improves this effect of MSCs themselves by improving microcirculation.

As described above, the medicament according to the present invention protects the vascular endothelium and suppresses endothelial dysfunction and thereby reduces risk of cerebral hemorrhage and enables a safe reperfusion therapy for vessel occlusion as defined in the appended claims, as well as has tissue repairing at infarction site, which is an original function of MSCs, and the effect that improves the motor function and ameliorates higher brain dysfunction, enabling whole new therapy for an ischemic vascular disorder.

### Examples

The present invention will be specifically described by the Examples below, but the present invention is not limited by these Examples.

### 1. Method

### (1) Preparation of mesenchymal stem cells derived from rat bone marrow

The experiment was conducted according to the Regulations for Animal Experiments at Sapporo Medical University. According to previous reports, the bone marrow obtained from thighbone of adult SD rats was diluted to 25 ml with Dulbecco's modified Eagle medium (DMEM), heat-inactivated 10% FBS, 2 mM 1-glutamine, 100 U/ml penicillin, and 0.1 mg/ml streptomycin were added, and the bone marrow was incubated for 3 days at 37 degrees Celsius in 5% CO₂ atmosphere (Kim S. et al., Brain Res. 2006, 1123:27-33. Ukai R. et al.,J. Neurotrauma. 2007, 24:508-520.). The bone marrow was cultured until confluent and adherent cells were detached with trypsin-EDTA and passaged at a density of 1 × 10⁴ cells/ml three times to obtain mesenchymal stem cells (MSCs).

### (2) Cerebral infarction model

As a cerebral infarction model, the rat transient middle cerebral artery occlusion (tMCAO) model was used. According to previous reports, adult male SD rats (280 to 330 g: n = 171) were anesthetized with ketamine (75 mg/kg) and xylazine (10 mg/kg) and a 20.0 to 22.0 mm of intraluminal suture (MONOSOF) was inserted from an external carotid artery to induce transient middle cerebral artery occlusion (Honma T. et al., Exp. Neurol. 2006; 199: 56-66. Sasaki M. et al., Methods Mol. Biol. 2009; 549: 187-195.).

### (3) Experimental protocol (Figure 1)

Sixty minutes after establishing transient middle cerebral artery occlusion, DWI-MRIs were obtained to evaluate the initial stroke volume. Animals with an initial stroke volume less than a standard (220 to 370 mm³) were excluded from the experiment. Thirty minutes after performing DWI-MRIs, the inserted intraluminal sutures were removed and the rats were randomized into four experimental groups as follows.

### Group 1 (physiological saline + medium, n = 43)

Rats were injected intravenously with normal saline (saline; 1ml) immediately after reperfusion, then injected fresh medium (DMEM: 1ml) after thirty minutes.

### Group 2 (rt-PA + medium, n = 48)

Rats were injected intravenously with rt-PA (10mg/kg; 1ml) immediately after reperfusion, then injected fresh medium (DMEM; 1 ml) after thirty minutes.

### Group 3 (physiological saline + MSC, n = 42)

Rats were injected intravenously with normal physiological saline (saline; 1ml) immediately after reperfusion, then injected MSCs (1.0 × 10⁶ cells each) in 1 ml fresh medium (DMEM) after thirty minutes.

### Group 4 (rt-PA + MSC, n = 38)

Rats were injected intravenously with rt-PA (10mg/kg; 1ml) immediately after reperfusion, then injected MSCs (1.0 × 10⁶ cells each) in 1 ml fresh medium (DMEM) after thirty minutes.

All rats were injected daily with cyclosporine A (10 mg/kg) intraperitoneally. All intravenous infusion was used through the left femoral vein.

### (4) MRI and measurement of ischemic lesion volume

Rats were anesthetized with ketamine (75 mg/kg) and xylazine (10 mg/kg) and MRI measurements were performed. The MRI measurements were performed using a 7-Teslar, 18-cm-bore superconducting magnet (Oxford Magnet Technologies) interfaced to a UNITYINOVA console (Oxford Instruments) as described previously (Honma T. et al., Exp. Neurol. 2006, 199:56-66., Komatsu K. et al., Brain Res. 2010, 1334:84-92.).

DWI were obtained sixty minutes after transient middle cerebral artery occlusion and 1, 4, 7, 14, 21 and 28 days after occlusion. The ischemia lesion area was calculated from the MRI image using Scion Image, Version Beta 4.0.2 (Scion Corporation). Lesion volume (mm³) was determined by analysis of high intensity areas on serial images collected through the cerebrum. For each slice, the higher intensity lesions in DWI and T2WI, where the signal intensity was 1.25 times higher than the counterpart in the contralateral brain lesion, were marked as the ischemic lesion area, and infarct volume was calculated taking slice thickness (1 mm) into account. The presence of intracerebral hemorrhage was counted when there is a low intensity area in the T2WI section. We used criteria for standardizing the initial stroke volume and excluded deviated animals before MSC or medium infusion.

Perfusion-weighted imaging (PWI) was acquired using T₂-weighted (TR=13□ms, TE=6.0□ms) gradient echo sequence. PWI measurements were obtained two hours after reperfusion of the middle cerebral artery occlusion. For cerebral blood flow (CBF) mapping by PWI, one coronal slice at Bregma -0.4mm was chosen for cerebral blood flow quantification. The regions of interests (ROI; 60 × 60 pixels) were placed in peri-infarcted lesion in the ischemic hemisphere. The coordinate relative to bregma for the center of ROI was on the DWI: 0.3 mm caudal. 1.25mm lateral. 0.1 mm depth. A regional cerebral blood flow (rCBF) in each ROI was quantified using a Perfusion Solver software. An rCBF ratio was calculated with the rCBF in the infarcted hemisphere divided by that of the non-infarcted hemisphere.

### (5) Gelatin zymography

Gelatin zymography was performed using brain tissue removed from rats at day1 after transient middle cerebral artery occlusion. Brain samples were homogenized immediately after perfusion in 10 times volume lysis buffer (150 mM NaCl, 1% sodium dodecyl sulfate [SDS], 0.1% deoxycholic acid, and 50 mM Tris-HCl, pH 7.5) containing protease inhibitors on ice and centrifuged for 15 minutes with centrifugal force: 9000 g, at four degrees Celsius, and then the supernatants were collected and stored at -80 degrees Celsius until use. Total protein concentration of each supernatant was determined by Thermo BCA protein assay (Pierce) and the activity of Matrix metalloproteinase (MMP)-9 was measured using a gelatin zymography kit (Primary Cell).

### (6) Treadmill stress test

Rats were trained 20 min/day for two days a week to run on a motor-driven treadmill (Muromachi Inc.) at a speed of 20 m/min with a slope of 20 before transient middle cerebral artery occlusion. The maximum speed at which the rats could run on a motor-driven treadmill was recorded at day 1, 4, 7, 14, 21 and 28 after transient middle cerebral artery occlusion.

### (7) Statistical analysis

All data presented is the average ± SEM, unless otherwise specified and statistical analysis was performed using JMP statistical discovery software (JMP10, SAS Institute Inc). Differences among groups were assessed by ANOVA with Tukey's post hoc test.

### 2. Results

### (1) Hemorrhage after rt-PA intravenous administration

Acute hemorrhagic events in Group 2 (rt-PA + medium) is higher than other three experimental groups in terms of incidence rate (Group 1 = 9.5%, Group 2 = 50%, Group 3 = 3.6%, Group 4 = 10%, p < 0.01, ANOVA: Figure 2B) and hemorrhagic volume (Group 1 = 0.14 +/- 0.01 mm³; Group 2 = 3.20 +/- 1.54 mm³; Group 3 = 0.01 +/- 0.01 mm³; Group 4 = 0.48 +/- 0.39 mm³, p < 0.01, ANOVA: Figure 2C). It should be noted that hemorrhagic events after rt-PA therapy in the group 2 were reduced by MSC infusion in the group 4 (incidence rate 40% decrease, p < 0.01, Tukey's post-hoc: 2.72 mm³ decrease in volume, p < 0.05, Tukey's post-hoc). This suggests that intravenous infusion MSCs has potential to reduce the incidence and volume of intracerebral hemorrhage.

### (2) Gelatin zymography

Gelatin zymography indicated the ipsilateral brain showed an MMP-9 activity in Group 4 (rt-PA + MSC) is lower than that of Group 2 (rt-PA + medium) (p < 0.01, Tukey's post-hoc) and an activation of MMP-9 in Group 3 (physiological saline + MSC) is lower than that of Group 1 (physiological saline + medium) (p < 0.01, Tukey's post-hoc: Figure 3). Although upregulation of MMP-9 contributes to hemorrhagic transformation after reperfusion, these results suggest MSC infusion might decease the activity of MMP-9 and suppress hemorrhagic events.

### (3) Ischemic lesion volume by MRI analysis

Ischemic lesion volume was evaluated in four test groups by using DWI-MRI. DWI-MRIs were obtained at sixty minutes (Column 1) and confirmed no difference of the initial stroke volume among the groups (Group 1 = 274 +/-33 mm³; n = 9, Group 2 = 279 +/- 27 mm³; n = 12, Group 3 = 267 +/- 38 mm³; n = 11, Group 4 = 276 +/- 38 mm³; n = 9, ANOVA, p = 0.75). T2WI-MRIs were obtained from the four groups at day 1 (Column 2), day 4 (Column 3), day 14 (Column 4), day 28 (Column 5) after transient middle cerebral artery occlusion (Figure 4A).

In all groups, estimated lesion volume gradually decreased over time between transient middle cerebral artery occlusion and day 28 (Figure 4B). The MSC treated groups displayed greater volume reduction as compared with medium infused groups at day 14, 21 and 28 (p < 0.01, Tukey's post-hoc: Figure of 4B). Lesion volume in the rt-PA + MSC group (group 4) is 22% at day 14, 21% at day 21 and 25% at day 28 smaller than the rt-PA-Medium group (Group 2), respectively.

Although there was no statistical significance, there were trends that the reduction in lesion volume was greatest for the rt-PA+MSC group (Group 4) at day 4, 7, 14, 21 and 28 and the ischemic lesion volume is highest for the rt-PA + Medium group (Group 2) over the time course until day 28 (Fig. 4B). These results indicate that the intravenous infusion of MSCs enhances reduction of ischemic lesion volume even if the rt-PA therapy is performed.

### (4) Analysis of cerebral blood flow

To assess rCBF, the PWI-derived CBF maps allowed further quantitative analysis for the hemodynamic changes of the defined lesion in the ischemic brain. At 1 hour after reperfusion of transient middle cerebral artery occlusion, the rCBF ratios are similar between the rt-PA therapy groups (Groups 2 and 4) and between the non-rt-PA therapy groups (Groups 1 and 3), but the rCBF ratios of the rt-PA therapy groups are approximately 23% higher than those of the non-rt-PA therapy groups (Figure 4C). These results suggest that rCBF ratio could be increased 23% by the rt-PA therapy with and without receiving intravenous infusion of MSCs following rt-PA therapy (p<0.01, ANOVA).

### (5) Behavioral function

The maximum speed at which the rats could run on a motor-driven treadmill was recorded. Before the transient middle cerebral artery occlusion, all rats reached at 70 m/min, but twenty-four hours after the transient middle cerebral artery occlusion, maximum velocity on the treadmill test was at its maximum deficit. Both the MSC infused groups (Group 3: n = 11, Group 4: n = 9) had greater maximum velocity from 4 to 28 days than non-MSC treated control (Group 1: n = 9, Group 2: n = 12), moreover, the Group 4 attained a higher velocity than the Group3 from day 7 to day 28. These results indicate that combination of intravenous administration of MSCs with rt-PA therapy promotes the postinfarction recovery in comparison with that with single administration of MSCs (Figure 5).

### 3. Discussion

Evaluation of cerebral hemorrhage incidence rate and hemorrhage volume after rt-PA therapy used in combination with intravenous administration of MSCs in an acute phase of transient middle cerebral artery occlusion indicated that the administration of MSCs can suppress vascular endothelial dysfunction. Although the rt-PA therapy group without MSCs showed higher incidence rate of intracerebral hemorrhage and larger stroke volume, intravenous infusion of MSCs with rt-PA therapy after transient middle cerebral artery occlusion results greater reduction in both the incidence rate and the ischemic lesion volume of cerebral hemorrhage.

The molecular mechanisms that underlie hemorrhagic events after rt-PA therapy remain unclear, however, previous studies suggested that MMP-9 is involved in disruption of vasculature in reperfusion after acute stroke.

MSCs protect the brain. Furthermore, the intravenous administration of MSCs might protect the disruption of vasculature by non-degradation of basal lamina and/or extracellular matrix with the reduced expression of MMP-9. Infused MSCs inhibits the endothelial dysfunction to decrease the incidence rate of both intracerebral hemorrhage and hemorrhagic volume even though rt-PA was infused in the acute phase of cerebral ischemia. Although therapeutic efficacy of MSCs are consistent with previous studies, the synergistic effects of rt-PA therapy and administration of MSCs in ischemic infarction, that is, the prominent improvement of function and decrease in ischemic lesion volume are remarkable results.

The rt-PA infused groups (Groups 2 and 4) showed increased rCBF in the lesion 2 hours after reperfusion. Group 2 exhibited rCBF increase, but no therapeutic effect by rt-PA administration. Therefore, even if the brain blood flow is improved, improved merely, there is no therapeutic effect. However, synergistic effects in therapeutic effect were found when it was combined with MSCs (Group 4). This therapeutic effect was higher in comparison with the single administration of MSCs (Group 3). Accordingly, the rt-PA therapy is considered to synergistically improve the therapeutic effect of MSCs by improving microcirculation and contribute to the improvement of the motor function and the amelioration of stroke lesion by MSCs.

The application of cell therapy is considered to many neurological diseases including cerebral infarction. The use of rt-PA is a currently established therapy within several hours after ischemic infarction. In the future, patients given rt-PA therapy in an acute phase will be clinically given MSCs. These research findings will support this protocol. Accordingly, the administration of MSCs reduces risk of cerebral hemorrhage by rt-PA. Furthermore, rt-PA therapy improves therapeutic effect of MSCs.

The transient cerebral ischemia model used in this Example is a model in which cerebral infarction is induced by inserting an intraluminal suture into a brain blood vessel and, after hemostasis for a certain time or longer, the blood flow was physically recanalized by removing the thread (physical removal). In Group 3 (physiological saline + MSC, n = 42) among the 4 groups, the cerebral hemorrhage suppressive effect of the administration of MSCs has been found, which indicates that MSCs have the cerebral hemorrhage suppressive effect also in a reperfusion therapy for vessel occlusion by physical removal of a blood clot. The method according to the present invention is also applicable to other methods of the physical removal including, for example, clot removal by intravascular therapy, mechanical clot removal therapy, carotid endarterectomy, and bypass surgery.

Furthermore, since t-PA (including rt-PA) has both effects as a platelet aggregation inhibitor and an anticoagulant agent, this Example suggests that MSCs have the cerebral hemorrhage suppressive effect even in reperfusion of occluded blood vessels by platelet aggregation inhibitors and anticoagulant agents.

### Industrial Availability

According to the present invention, risk of cerebral hemorrhage associated with a reperfusion therapy for vessel occlusion comprising administration of a tissue plasminogen activator) can be reduced and therapeutic effect on ischemic vascular disorders can be improved. The present invention is useful in therapy for ischemic vascular disorders including cerebral infarction and myocardial infarction.

## Claims

1. A medicament comprising mesenchymal stem cells for use in the treatment of an ischemic vascular disorder, wherein the medicament is used in combination with a reperfusion therapy for vessel occlusion,
wherein the reperfusion therapy for vessel occlusion comprises administration of a tissue plasminogen activator.

2. The medicament for use according to claim 1, wherein the medicament reduces risk of cerebral hemorrhage associated with the reperfusion therapy for vessel occlusion.

3. The medicament for use according to claim 1 or 2, wherein the ischemic vascular disorder is an ischemic cerebrovascular disorder or myocardial infarction, preferably, wherein the ischemic vascular disorder is a super acute phase or acute phase of an ischemic cerebrovascular disorder or acute myocardial infarction.

4. The medicament for use according to any one of claims 1 to 3, wherein the mesenchymal stem cells are mesenchymal stem cells derived from bone marrow.

5. A medicament comprising mesenchymal stem cells for use in the prevention of cerebral hemorrhage associated with a reperfusion therapy for vessel occlusion, wherein the reperfusion therapy for vessel occlusion comprises administration of a tissue plasminogen activator.

6. The medicament for use according to claim 5, wherein the medicament is used for a patient with an ischemic vascular disorder.

7. The medicament for use according to claim 6, wherein the ischemic vascular disorder is an ischemic cerebrovascular disorder or myocardial infarction, preferably, wherein the ischemic vascular disorder is a super acute phase or acute phase of an ischemic cerebrovascular disorder or acute myocardial infarction.

8. The medicament for use according to any one of claims 5 to 7, wherein the mesenchymal stem cells are mesenchymal stem cells derived from bone marrow.

## Patentansprüche

1. Mesenchymale Stammzellen umfassendes Medikament zur Verwendung bei der Behandlung einer ischämischen vaskulären Störung, wobei das Medikament in Kombination mit einer Reperfusionstherapie für Gefäßverschluss verwendet wird, wobei die Reperfusionstherapie für Gefäßverschluss die Verabreichung eines Gewebe-Plasminogenaktivators umfasst.

2. Medikament zur Verwendung gemäß Anspruch 1, wobei das Medikament das Risiko von mit der Reperfusionstherapie für Gefäßverschluss assoziierter Hirnblutung reduziert.

3. Medikament zur Verwendung gemäß Anspruch 1 oder 2, wobei die ischämische vaskuläre Störung eine ischämische zerebrovaskuläre Störung oder ein Myokardinfarkt ist, wobei die ischämische vaskuläre Störung bevorzugt eine superakute Phase oder eine akute Phase einer ischämischen zerebrovaskulären Störung oder ein akuter Myokardinfarkt ist.

4. Medikament zur Verwendung gemäß irgendeinem der Ansprüche 1 bis 3, wobei die mesenchymalen Stammzellen von Knochenmark abgeleitete mesenchymale Stammzellen sind.

5. Mesenchymale Stammzellen umfassendes Medikament zur Verwendung bei der Prävention von mit einer Reperfusionstherapie für Gefäßverschluss assoziierter Hirnblutung, wobei die Reperfusionstherapie für Gefäßverschluss die Verabreichung eines Gewebe-Plasminogenaktivators umfasst.

6. Medikament zur Verwendung gemäß Anspruch 5, wobei das Medikament für einen Patienten mit einer ischämischen vaskulären Störung verwendet wird.

7. Medikament zur Verwendung gemäß Anspruch 6, wobei die ischämische vaskuläre Störung eine ischämische zerebrovaskuläre Störung oder ein Myokardinfarkt ist, wobei die ischämische vaskuläre Störung bevorzugt eine superakute Phase oder eine akute Phase einer ischämischen zerebrovaskulären Störung oder ein akuter Myokardinfarkt ist.

8. Medikament zur Verwendung gemäß irgendeinem der Ansprüche 5 bis 7, wobei die mesenchymalen Stammzellen vom Knochenmark abgeleitete mesenchymale Stammzellen sind.

## Revendications

1. Médicament comprenant des cellules souches mésenchymateuses destiné à être utilisé dans le traitement d'un accident vasculaire ischémique,
dans lequel le médicament est utilisé en association avec un traitement de reperfusion d'une occlusion vasculaire,
dans lequel le traitement de reperfusion d'une occlusion vasculaire comprend l'administration d'un activateur tissulaire du plasminogène.

2. Médicament destiné à être utilisé selon la revendication 1, dans lequel le médicament diminue le risque d'hémorragie cérébrale lié au traitement de reperfusion d'une occlusion vasculaire.

3. Médicament destiné à être utilisé selon la revendication 1 ou 2, dans lequel l'accident vasculaire ischémique est un accident vasculaire cérébral ischémique ou un infarctus du myocarde, de préférence, dans lequel l'accident vasculaire ischémique est un accident vasculaire cérébral ischémique en phase très aiguë ou en phase aiguë ou un infarctus du myocarde aigu.

4. Médicament destiné à être utilisé selon l'une quelconque des revendications 1 à 3, dans lequel les cellules souches mésenchymateuses sont des cellules souches mésenchymateuses provenant de la moelle osseuse.

5. Médicament comprenant des cellules souches mésenchymateuses destiné à être utilisé en prévention d'une hémorragie cérébrale liée à un traitement de reperfusion d'une occlusion vasculaire, dans lequel le traitement de reperfusion d'une occlusion vasculaire comprend l'administration d'un activateur tissulaire du plasminogène.

6. Médicament destiné à être utilisé selon la revendication 5, dans lequel le médicament est utilisé pour un patient présentant un accident vasculaire ischémique.

7. Médicament destiné à être utilisé selon la revendication 6, dans lequel l'accident vasculaire ischémique est un accident vasculaire cérébral ischémique ou un infarctus du myocarde, de préférence, dans lequel l'accident vasculaire ischémique est un accident vasculaire cérébral ischémique en phase très aiguë ou en phase aiguë ou un infarctus du myocarde aigu.

8. Médicament destiné à être utilisé selon l'une quelconque des revendications 5 à 7, dans lequel les cellules souches mésenchymateuses sont des cellules souches mésenchymateuses provenant de la moelle osseuse.
